# FASCICULE DE BREVET EUROPEEN

(11) **EP 1 807 187 B1**
(45) Date de publication et mention de la délivrance du brevet: **08.05.2019**
(21) Numéro de dépôt: 05805587.2
(22) Date de dépôt: 08.09.2005
(51) Int. Cl.: B01J 2/00

(54) **PROCEDE D'ENROBAGE DE POUDRES**
VERFAHREN ZUR PULVERBESCHICHTUNG
METHOD FOR COATING POWDERS

(30) Priorité: 09.09.2004 FR 0409563
(43) Date de publication de la demande: 18.07.2007
(73) Titulaire: PIERRE FABRE MEDICAMENT, 92100 Boulogne-Billancourt (FR)
(72) Inventeur: CALDERONE, Marilyn, F-34090 Montpellier (FR); RODIER, Elisabeth, F-81430 Marsal (FR)
(74) Mandataire: Regimbeau
(86) Numéro de dépôt international: PCT/FR2005/002236
(87) Numéro de publication internationale: WO 2006/030112

(56) Documents cités:
- WO-A-98/15348
- DE-A1- 10 233 864
- FR-A- 2 838 356
- US-A- 5 509 959
- US-A1- 2001 000 143
- US-A1- 2004 079 459

## Description

Le domaine de l'invention concerne l'enrobage de particules de substances actives solides en particulier de taille inférieure à 100µm et avantageusement inférieure à 50µm par un procédé utilisant un fluide supercritique ou subcritique et donc la fabrication de microcapsules.

L'état de l'art sur la technique d'enrobage au niveau industriel comprend des procédés chimiques ou mécaniques, c'est à dire n'incluant que des phénomènes physiques (J. Richard et al, Techniques de l'ingénieur, Microencapsulation, J 2 210, 1-20, 2002) et des procédés physico-chimiques.
Les procédés chimiques sont basés sur la formation in situ du matériau enrobant par polycondensation, polymérisation, etc. Les procédés mécaniques mettent en oeuvre des techniques de pulvérisation (spray drying, spray coating), de formation de gouttes ou gouttelettes (prilling) et d'extrusion (extrusion, sphéronisation). Enfin, les procédés physico-chimiques sont basés sur la maîtrise de la variation de la solubilité et des conditions de précipitation des agents enrobants : pH, température, etc.
On connaît parmi les procédés physico-chimiques, les procédés de coacervation, qui sont limités aux seuls principes actifs lipophiles. On connaît aussi les procédés d'évaporation ou d'extraction de solvant, faciles à mettre en oeuvre. Les principales limites de ces procédés sont qu'ils ne s'appliquent qu'à l'encapsulation de matière active lipophile, et surtout qu'ils mettent en jeu des solvants organiques. D'une part, les produits obtenus ont une teneur en solvant résiduel non négligeable et d'autre part, une étape de séchage est nécessaire pour finaliser l'enrobage des particules.

Enfin parmi les procédés physico-chimiques, on trouve aussi la microencapsulation par coacervation simple et par gélification thermique. Le premier procédé met aussi en oeuvre des solvants organiques et le second a quant à lui, pour principal inconvénient d'être limité aux principes actifs qui ne sont pas thermosensibles.
On connaît aussi pour l'enrobage, les procédés chimiques, comme la polycondensation interfaciale, la polymérisation en émulsion, en suspension, en dispersion, etc qui permettent de fabriquer in situ une membrane de polymère à la surface de gouttelettes d'émulsion, grâce à une réaction chimique entre deux monomères. La réaction chimique se déroule à l'interface entre la phase dispersée et la phase dispersante. Cette méthode d'enrobage s'applique seulement à des solutions de matières actives. Elle n'est donc pas applicable à l'enrobage des formes solides.
L'enrobage de formes solides s'effectue suivant des procédés que l'on peut classer en deux sous-groupes, les procédés mécaniques et les procédés utilisant la technique des fluides supercritiques.
Les procédés mécaniques sont basés sur une pulvérisation (spray-drying ou spray-coating, fluidisation), une formation de gouttes (prilling) ou sur un principe d'extrusion. Les procédés utilisant une pulvérisation ou la formation de gouttes passent par l'emploi d'une formulation liquide constituée d'un agent enrobant dissous dans un solvant. L'enrobé peut être sous forme liquide ou solide. Le principe d'extrusion met en jeu un milieu fondu constitué de l'agent enrobant fondu mélangé avec un enrobé solide ou fondu qui doit être stable thermiquement à la température d'extrusion généralement comprise entre 70 °C et 150 °C. La taille des microcapsules obtenues par ces procédés est généralement supérieure ou égale à 100 µm. Ces procédés ne s'avèrent donc pas adaptés à l'enrobage des enrobés sous formes solides dont la taille est inférieure à 100 microns et surtout aux enrobés thermosensibles.
Les procédés utilisant un gaz comprimé sont basés le plus souvent sur l'emploi du CO₂ supercritique. En effet, celui-ci présente de nombreux avantages : des coordonnées critiques relativement basses (31°C, 7,47 MPa), une grande variation de son pouvoir solvant pour de faibles variations de pressions, sa non toxicité, son coût peu onéreux. Les trois grandes mises en oeuvre en vue d'un enrobage de poudres sont la technique RESS (Rapid Expansion of Supercritical Solution), la technique PGSS (Particles from Gas Saturated Solution), et la technique SAS (Supercritical Anti Solvent).
La technique RESS (Türk et al, Journal of Supercritical Fluids, 15, 1999, 79-89) permet d'enrober des particules dont la taille granulométrique peut descendre actuellement jusqu'à 50 microns en utilisant la technologie des lits fluidisés par un fluide supercritique. Ce procédé maîtrisé par l'équipe allemande de Brunner (Journal of Supercritical Fluids, 24, 2002, 137-151), consiste à fluidiser les particules que l'on veut enrober, par un fluide en conditions supercritiques, puis à détendre dans ce lit au moyen d'une buse de pulvérisation, sur les particules fluidisées, une phase supercritique saturée en enrobant. L'extraction de l'enrobant par le fluide supercritique a lieu dans une enceinte distincte du lit fluidisé. La différence de pression entre l'enceinte d'extraction et le lit fluidisé provoque une chute brutale de la sursaturation et par la même, la cristallisation de l'agent d'enrobage. Cette technique prometteuse reste cependant limitée aux seuls enrobants solubles dans le CO₂ supercritique. La gamme d'enrobants envisageables reste donc très restreinte.

En revanche, la solubilité du CO₂ comprimé dans les liquides et les solides est généralement très élevée. Ce principe a donné naissance au procédé PGSS. Le fluide supercritique ou subcritique n'est donc pas ici comme pour le procédé RESS le solvant mais le soluté. Le principal avantage du procédé PGSS est donc d'élargir considérablement le panel des enrobants que l'on peut travailler avec un gaz comprimé. La technique PGSS mise en oeuvre dans WO 02/05944 et US 6 056 791, repose sur la solubilisation de CO₂ comprimé dans l'agent enrobant dont on abaisse ainsi le point de fusion ou la température de transition vitreuse. La solubilisation du gaz comprimé dans l'enrobant provoque son ramollissement puis la détente de cette phase ramollie engendre la solidification de ce matériau sous forme de solide pulvérulent. Pour l'enrobage de particules, l'enrobé est mélangé à ce milieu fondu. L'ensemble enrobé/agent d'enrobage est ensuite détendu, ce qui provoque la solidification de l'agent enrobant sur l'enrobé. Une lacune majeure de ce procédé d'enrobage réside dans le fait qu'il n'est pas adapté aux principes actifs thermosensibles puisque l'enrobant et l'enrobé sont mis dans des conditions de pression et température similaires pendant toute la durée, généralement longue, de la solubilisation du gaz comprimé dans l'enrobant. D'autre part, la maîtrise exacte du ratio enrobé / enrobant s'avère bien souvent difficile.
D'autre part, on connaît aussi le procédé CPF Technology (Concentrated Powder Form) reposant sur le principe PGSS (WO99/17868). Il consiste à co-pulvériser un principe actif liquide mis au préalable sous pression avec un auxiliaire pulvérulent pour obtenir des formulations solides / liquides. Un gaz sous pression est dissous dans une solution de principe actif jusqu'à saturation. Cette solution saturée est rapidement détendue à travers une buse formant ainsi des très fines gouttelettes. Pendant l'expansion une poudre de stabilisant est additionnée à co-courant du spray liquide. L'expansion du gaz en sortie de buse provoque un mélange intensif entre les gouttes de liquide issues de l'aérosol et le solide pulvérulent additionné. Le liquide du spray est alors adsorbé à la surface du solide et des particules solides / liquides sont formées. Le procédé CPF permet ainsi de convertir une forme liquide en solide par adsorption sur un support solide. Une des principales applications concerne l'amélioration du transport et du stockage des particules composites solides / liquides par rapport à la forme liquide de départ. Cette technologie permet aussi d'obtenir des particules composites originales solides / liquides selon les propriétés d'adsorption du solide support. Le but recherché ici est de stabiliser un principe actif sous forme liquide par un produit pulvérulent.
La technique SAS appliquée à l'enrobage de principes actifs (brevets US 5 043 280 et FR 2 753 639) exige au préalable la solubilisation de l'agent enrobant dans un solvant organique.

L'enrobé est dispersé dans cette solution. Cette dispersion est ensuite co-injectée avec du CO₂ supercritique. Ce dernier joue le rôle d'antisolvant et permet ainsi de solidifier l'agent enrobant sur l'enrobé. Une des contraintes majeures du procédé réside dans l'emploi d'un solvant organique, qu'il faut ensuite séparer de l'antisolvant mais aussi et surtout dans le fait que le principe actif reste pendant toute la durée du procédé dans des conditions de pression et température bien souvent élevées (et au minimum au delà de 31 °C si on utilise le CO₂ comme fluide supercritique).
Foster et al. (Powder Technologie, 126, 2002, 134-149) présentent une mise en oeuvre spécifique du procédé antisolvant par le procédé ASES (Aérosol Solvant Extraction System). Une buse de pulvérisation pour l'enrobage de principes actifs permet de déposer de l'enrobant sur les particules formées simultanément par effet antisolvant. En effet, la buse développée comporte trois sections annulaires concentriques permettant d'introduire les divers fluides dans une enceinte en conditions supercritiques. Au centre est véhiculée la solution de principe actif dissous dans un solvant organique. La partie centrale de la buse permet l'acheminement de la solution organique avec le polymère dissous et la partie externe de la buse permet de véhiculer le fluide supercritique qui produit l'effet antisolvant. Ainsi l'enrobant se dépose de façon préférentielle sur le principe actif pour l'enrober. L'enrobant et l'enrobé cristallisent simultanément. On notera que ce type de mise en contact enrobé / enrobant est réalisé sur des courants de fluides et la buse ne véhicule à aucun moment le principe actif sous forme solide.
On peut également citer la demande internationale WO 98/15348, qui concerne un procédé de micro-encapsulation de matériau de coeur comprenant les étapes consistant à a) mélanger un matériau de coeur avec un polymère d'encapsulation, b) fournir au mélange un fluide supercritique capable de faire gonfler le polymère à une température et une pression suffisantes pour maintenir le fluide dans un état supercritique, c) permettre au fluide supercritique de pénétrer et de liquéfier le polymère tout en maintenant la température et la pression suffisantes pour maintenir le fluide dans un état supercritique, et d) libérer rapidement la pression pour solidifier le polymère autour du matériau de coeur pour former une microcapsule. Dans ce procédé, l'étape de mise en contact de particules avec le mélange se fait simultanément à l'étape de détente et d'enrobage.
Mention peut aussi être faite du brevet allemand DE 102 33 864 décrit un procédé d'enrobage de particules où les particules sont mélangées dans le mélange d'enrobage qui est soumis à la détente en présence de CO2 supercritique.
Le document US 5 509 959 décrit une composition de précurseurs d'enrobage adaptée pour être mélangée avec un fluide supercritique utilisé en tant que diluant et réduisant sa viscosité, l'ensemble liquide en résultant étant ensuite pulvérisé sur un substrat, qui n'est pas une substance active. Toutefois, dans ce document, l'état du substrat, à enrober (particules individualisées ou non) n'est pas précisé.
La demande US 2004/079459 concerne un procédé de rechapage d'un pneumatique comprenant l'étape consistant à utiliser un fluide supercritique en tant que support pour pulvériser une couche de coussin de gomme sur la surface extérieure (radialement à l'extérieur) de la carcasse d'un pneumatique.
La demande FR2838356 concerne un procédé d'obtention d'une suspension de particules solides d'au moins un produit. Ce procédé est caractérisé en ce qu'il comporte les étapes consistant à mettre en solution le produit au sein d'un fluide comprimé, détendre cette solution par un moyen de décompression à travers lequel est pulvérisé simultanément, un liquide au sein duquel le produit est insoluble ou très peu soluble, et collecter les particules ainsi formées en suspension dans le liquide.
La demande de brevet US 2001/0000143 décrit un procédé d'obtention d'un principe actif, préférentiellement un caroténoïde, sous forme de poudre. Ce procédé comprend la mise sous pression à haute température (40°C-150°C) de ce principe actif en présence de diméthyléther aboutissant à la solubilisation du principe actif dans le gaz. La pression est ensuite relâchée, provoquant la pulvérisation du principe actif sous forme de poudre. Ce procédé donc n'est pas applicable à des principes actifs thermosensibles. Ce procédé aboutit à la formation de particules de principe actif incluses dans une matrice, et non pas à des particules individualisées enrobées comprenant un noyau de principe actif et une écorce d'enrobant. En outre, le matériau solide ainsi obtenu contient une quantité résiduelle de solvant organique (diméthyléther), bien que réduite par rapport aux procédés connus jusqu'alors.
Enfin l'inventaire des brevets d'enrobage par voie supercritique ne saurait être complet si l'on omet de mentionner la demande de brevet FR 2 809 309. Elle concerne la formation de microsphères de type matricielles destinées à être injectées, comprenant un principe actif protéique et un agent enrobant ce principe actif. L'enrobage vise à prolonger la libération. Ledit agent d'enrobage doit être soluble dans le fluide supercritique. Les particules enrobées obtenues sont caractérisées en ce qu'elles sont dépourvues de solvant organique. Le principe de ce procédé consiste à placer dans un autoclave muni d'une agitation le principe actif et l'agent d'enrobage, puis à introduire sous agitation, un fluide en conditions supercritiques, de façon à ce que celui-ci dissolve l'enrobant. Le principe actif reste solide tout au long du procédé. Une fois l'enrobant dissous, la température et la pression de la suspension sont réduites de façon lente et contrôlée pour faire chuter la sursaturation de l'enrobant dans la phase supercritique. L'agent d'enrobage se dépose alors de façon préférentielle sur le principe actif et forme une couche protectrice d'enrobage. Ce procédé reste toutefois limité aux enrobants présentant une solubilité suffisante en présence d'un fluide supercritique.

Aux vues des différentes techniques citées, il apparaît nécessaire de disposer d'un procédé d'enrobage de particules solides de substances actives de taille plus réduite et thermosensibles par une gamme d'enrobants plus large et en particulier par des enrobants qui ne sont pas solubles dans les fluides supercritiques ou subcritiques.

A cette fin, la présente invention concerne un procédé d'enrobage de substances actives solides pulvérulentes caractérisé en ce qu'il comprend les étapes suivantes :
a) fournir un mélange comprenant au moins un enrobant dans lequel est dissous jusqu'à saturation un fluide supercritique ou subcritique dans des conditions de température et de pression permettant le maintien dudit fluide dans des conditions supercritiques ou subcritiques ;
b) fournir des particules individualisées en mouvement d'au moins une substance active solide pulvérulente ;
c) mettre en contact le mélange et les particules individualisées en mouvement de substance active dans des conditions de température et de pression assurant simultanément :
   - la détente du fluide supercritique ou subcritique et son retour à l'état gazeux,
   - la pulvérisation de l'enrobant et
   - sa solidification autour des particules individualisées en mouvement de substance active ;
d) séparer et récupérer les particules enrobées.

Un des avantages du procédé selon la présente invention réside dans le fait que grâce à la mise en oeuvre de températures d'environ 25°C et donc, proches de la température ambiante, le procédé selon la présente invention est particulièrement adapté à l'enrobage de substances actives thermosensibles : en effet, d'une part la substance active et l'enrobant sont traités de façon séparée, et d'autre part la durée de mise en contact entre la substance active et l'enrobant est très brève, les caractéristiques de l'enrobant pouvant en outre être modifiées lors de l'étape (a) (abaissement de la température de fusion par exemple).

Le procédé tel que décrit par la présente invention permet également une bonne maîtrise du ratio massique substance active / enrobant dans la gamme pouvant être comprise entre 10/1 et 1/10, avantageusement entre 5/1 et 1/5 et donc des quantités de substances actives et d'enrobant mises enjeu.
Enfin, puisque le principe du procédé selon la présente invention repose sur la solubilisation d'un fluide supercritique ou subcritique dans l'enrobant et non sur la dissolution de l'enrobant par ledit fluide, il est applicable à un large panel d'enrobants et en particulier à des enrobants non solubles dans ledit fluide.

Par le terme d'« enrobage », on entend au sens de la présente invention le dépôt d'une couche continue d'un enrobant sur une particule de substance active solide. On obtient ainsi une structure de type noyau solide / écorce solide continue. L'écorce peut être poreuse ou non. Les particules obtenues sont donc des microcapsules.

Par le terme de « substance active », on entend au sens de la présente invention tout principe actif pharmaceutique (antalgiques, antipyrétiques, aspirine et dérivés, antibiotiques, anti-inflammatoires, anti-ulcéreux, anti-hypertenseurs, neuroleptiques, anti-dépresseurs, oligonucléotides, peptides, protéines par exemple) ; cosmétique (anti UV, autobronzant par exemple), nutraceutique (vitamines par exemple) ; alimentaire ; agrochimique ou vétérinaire. Avantageusement, il s'agit de la pseudoéphédrine. Avantageusement la substance active est thermosensible. Par « substance active thermosensible », on entend au sens de la présente invention toute substance active qui sous l'action de la température voit sa structure physico-chimique modifiée. Avantageusement, ces substances sont sensibles à des températures supérieures à environ 28°C, de façon avantageuse supérieures à environ 25°C. Avantageusement, il s'agit des protéines ou de peptides, avantageusement de la BSA (Bovin Serum Albumin).
Par le terme d'« enrobant », on entend au sens de la présente invention un ou plusieurs matériau(x) de revêtement déposés à la surface de la substance active et capable de former une couche continue solide autour de la particule solide de substance active. De façon avantageuse, l'enrobant n'est pas soluble dans le fluide supercritique ou subcritique. On utilisera avantageusement des corps gras tels que des phospholipides, notamment la phosphatidylcholine, le phosphatidylglycérol, le diphosphatydylglycérol, la dipalmitoylphosphatidylcholine, la dioeylphosphatydyéthanolamine, des triglycérides des acides capriques et capryliques, des esters d'acides gras solides, notamment leurs esters d'acides gras de C₈ à C₁₈ comme le palmitate d'éthyle, le myristade d'éthyle, le myristade d'octyldodécyle, avantageusement les esters d'acides gras en C₈ à C₁₈ et leurs mélanges. Il peut aussi s'agir des polysaccharides et de leurs dérivés tels que l'amidon ou l'amidon modifié comme les carboxyméthylamidons; la cellulose ou l'éthylcellulose modifié comme les carboxyméthylcelluloses, l'éthylcellulose, l'hydroxyéthylcellulose, l'hydroxypropylméthylcellulose, la méthylhydroxyéthylcellulose, la méthylhydroxypropylcellulose ; les polysaccharides issus d'alginate, de carraghénanes, de pectine, de pectates, de guars, de xanthanes et de chitosan ; des polymères synthétiques de type acryliques ou méthacryliques comme les homopolymères ou les copolymères de l'acide acrylique ou méthacrylique, le polyacrylamide, les polycyanoacrylates, et en général tous les polymères synthétiques bien connus de l'homme du métier dérivés de l'acide acrylique ou méthacrylique, des polymères et copolymères vinyliques dérivés des esters vinyliques (polyacétate de vinyle), les copolymères de l'éthylène et de l'acétate de vinyle ; des polymères et copolymères biodégradables des acides α-hydroxycarboxyliques, notamment les homopolymères et copolymères des acides lactiques et glycoliques, plus particulièrement le PLA et le PLGA ; du poly(ε-caprolactone) et ses dérivés, les poly(β-hydroxybutyrate), poly(hydroxyvalérate) et les copolymères β-hydroxybutyrate-hydroxyvalérate, le polyacide malique ; des polymères blocs amphiphiles de type polyacide lactique, polyoxyde d'éthylène, les polymères biocompatibles de type polyéthylène glycol, polyoxydes d'éthylène, les copolymères blocs de type polyoxyde d'éthylène, polyoxyde de propylène ; les polyanhydrides, les polyorthoesters, les polyphosphazènes et leurs mélanges. Avantageusement l'enrobant est choisi dans le groupe comprenant les polysaccharides et leurs dérivés, les polymères synthétiques de type acryliques ou méthacryliques, les lipides, les phospholipides ou un mélange de ceux-ci.
Avantageusement, l'enrobant est choisi parmi un mélange de mono, di et triglycérides, avantageusement un dipalmitostéarate de glycérol tel que le Précirol® commercialisé par Gattefossé, la paraffine et le monostéarate de glycérol.
Avantageusement, l'enrobant permet de masquer le goût et/ou la couleur de la substance active.
De façon avantageuse, l'enrobant permet une libération contrôlée de la substance active et une augmentation de sa biodisponibilité.
De façon encore plus avantageuse l'enrobant protège la substance active vis à vis d'agents externes de dégradation.

Avantageusement dans ce cas, la substance active est thermosensible et/ou chemosensible et/ou photosensible.

Par le terme de « fluide supercritique ou subcritique », on entend au sens de la présente invention tout fluide utilisé à une température ou une pression supérieure à leur valeur critique ou à une température ou une pression inférieure mais proche de leur valeur critique. Dans la présente invention, il s'agit du CO₂, gaz susceptible de saturer l'enrobant.

Le procédé d'enrobage de la présente invention est un procédé dit « mécanique » puisqu'il met en oeuvre une technique de pulvérisation de l'enrobant sur les particules individualisées en mouvement de substance active. Ledit procédé concerne l'enrobage d'une substance active sous forme solide pulvérulente par un enrobant, liquéfié par un fluide supercritique ou subcritique dans des conditions de température et de pression permettant le maintien dudit fluide dans des conditions supercritiques ou subcritiques, puis solidifié par la détente. La substance active et l'enrobant, sont donc tous deux sous forme solide dans les particules finales obtenues par le procédé selon la présente invention.
Le procédé de la présente invention implique donc deux étapes principales: la solubilisation d'un fluide supercritique ou subcritique dans un enrobant puis la détente du mélange enrobant / fluide supercritique ou subcritique lors de la mise en contact simultanée avec les particules individualisées en mouvement de substance active. On peut également envisager une mise en conditions supercritiques ou subcritiques du fluide après le mélange dudit fluide avec l'enrobant.
De plus, un solvant organique choisi dans le groupe comprenant des cétones, alcools, esters, alcanes, les alcènes ou un mélange de ceux-ci peut être ajouté aux particules individualisées en mouvement de substance active et/ou au mélange enrobant/fluide supercritique ou subcritique.
En effet, on peut envisager d'enrober des nanopoudres fabriquées in situ par CO₂ supercritique. Dans ce cas, le procédé nécessite l'adjonction d'un co-solvant. Les particules à enrober sont alors mises en solution dans ledit co-solvant, et la solution est mise en contact avec l'enrobant et le fluide supercritique ou subcritique.
Par ailleurs, l'ajout d'un solvant en faible quantité permet de modifier la solubilité du CO₂ dans l'agent enrobant, et il est donc possible de modifier les débits du mélange obtenu en préservant les mêmes caractéristiques d'enrobage (ratio substance active / enrobant).
De même un agent plastifiant peut être ajouté au mélange enrobant/fluide supercritique ou subcritique. Le plastifiant permet d'améliorer les qualités de surface de l'enrobant selon l'application désirée.
La première étape permet de modifier les caractéristiques physico-chimiques de l'enrobant par exemple en abaissant le point de fusion, la température de transition vitreuse et la viscosité. La deuxième étape implique la détente du mélange comprenant l'enrobant fondu ou ramolli et le fluide pour former un aérosol : produit sous forme micronisée. L'abaissement de température et de pression ainsi généré permet de solidifier l'enrobant autour des particules individualisées de substance active solide en mouvement, ces particules de substance active pouvant être éventuellement déjà présentes en mouvement dans l'enceinte où est détendu le mélange enrobant / fluide ou transportées et injectées simultanément à la détente dans cette même enceinte où a lieu la mise en contact.
En fin de procédé, les particules enrobées peuvent être récupérées dans un filtre de séparation gaz / solide.
Une caractéristique fondamentale du procédé de la présente invention réside dans la fluidisation des particules de substance active à enrober qui permet de présenter ladite substance sous forme de fines particules individualisées, non agglomérées, en mouvement et d'enrober ainsi lesdites particules de substance active individualisées en mouvement de taille inférieure à 100µm et avantageusement inférieure à 50µm, de façon encore plus avantageuse inférieure à 20µm, et non des agrégats de matière active. Cette mise en mouvement des particules de substance active peut être obtenue de différentes manières connues de l'homme du métier : pneumatique, mécanique ou par lit fluidisé par exemple.

Dans un mode particulier de réalisation de l'invention, les particules individualisées de substance active sont convoyées par transport pneumatique ou mécanique (avec du CO₂ ou de l'air comprimé par exemple). Ce type de convoyage permet en effet de fluidiser les particules que l'on va enrober et d'en éviter l'agrégation. Dans le cas d'un transport pneumatique, on utilisera de préférence comme gaz le CO₂. Avantageusement, ce CO₂ pourra être recyclé pour permettre une réutilisation en continu des gaz, réutilisation possible dans le cas où le fluide supercritique ou subcritique solubilisé dans l'enrobant est aussi du CO₂.

Dans un mode particulier de réalisation de l'invention, l'étape (c) de mise en contact du mélange avec les particules individualisées de substance active est réalisée dans un courant transporteur obtenu par co-injection. Avantageusement, cette co-injection consiste en l'injection simultanée au travers du tube co-axial des particules individualisées de substance active à enrober et du mélange enrobant / fluide.
De façon avantageuse, le courant transporteur est linéaire ou hélicoïdal.

Le procédé selon la présente invention est également utilisable dans le cas d'enrobage de plusieurs substances actives différentes destinées à une même formulation finale.

Le procédé selon la présente invention s'applique à des particules de substance active dont la forme géométrique peut être variée (forme régulière: sphérique ou certaines formes cristallines par exemple ; ou toute autre forme irrégulière).

La présente invention concerne en outre des microparticules de substance active enrobées, chaque microparticule comprenant successivement un noyau comprenant la substance active et une écorce comprenant l'enrobant, caractérisées
- en ce qu'elles sont susceptibles d'être obtenues par le procédé selon la présente invention,
- en ce que la substance active est solide à température ambiante et thermosensible,
- en ce que la taille moyenne du noyau de substance active est inférieure à 50µm, avantageusement inférieure à 20µm.

Avantageusement, l'enrobant est solide à température ambiante et n'est pas soluble dans le fluide supercritique ou subcritique. De façon avantageuse, les particules selon la présente invention sont exemptes de solvant organique.

La présente invention concerne en outre des particules de substance active enrobées, chaque particule comprenant successivement un noyau comprenant la substance active et une écorce comprenant l'enrobant, caractérisées
- en ce qu'elles sont susceptibles d'être obtenues par le procédé selon la présente invention,
- en ce que la substance active est solide à température ambiante et thermosensible,
- en ce que la taille moyenne du noyau de substance active est inférieure à 50µm,
- en ce que l'enrobant est solide à température ambiante et n'est pas soluble dans le fluide supercritique ou subcritique et
- en ce que les particules enrobées sont exemptes de solvant organique.

Avantageusement l'enrobant permet de masquer le goût et/ou la couleur de la substance active et/ou permet une libération contrôlée de la substance active et une augmentation de sa biodisponibilité et/ou permet de protéger la substance active vis à vis d'agents externes de dégradation. Avantageusement, la substance active est chemosensible et/ou photosensible. De façon avantageuse, l'enrobant permet de formuler deux ou plusieurs substances actives de nature incompatibles entre elles et ainsi de les protéger les unes par rapport aux autres.

La présente invention concerne en outre l'utilisation des particules selon la présente invention dans une formulation orale, topique, injectable ou rectale et/ou dans des formulations pharmaceutiques, cosmétiques, nutraceutiques, alimentaires, agrochimiques ou vétérinaires.

L'invention sera mieux comprise et les buts, avantages et caractéristiques de celle-ci apparaîtront plus clairement de la description qui suit et qui est faite en référence aux dessins annexés représentant des exemples non limitatifs de réalisation de l'invention sur lesquels :
La figure 1 représente un premier mode de mise en oeuvre du procédé selon la présente invention avec recirculation des particules individualisées de substance active non enrobées.
La figure 2 représente un deuxième mode de mise en oeuvre du procédé selon la présente invention avec recirculation du CO₂ assurant les deux fonctions de fluide supercritique ou subcritique dissous dans l'enrobant au niveau de l'autoclave et de gaz vecteur auprès des particules individualisées de substance active à enrober.
Les figures 3 et 4 représentent un mode de co-injection des particules individualisées en mouvement substance active et du mélange enrobant/fluide supercritique ou subcritique dans des tubes concentriques.
La figure 5 représente le pourcentage volumétrique différentiel obtenu pour des particules de verre non enrobées ou enrobées par le procédé selon la présente invention.
Les figures 6, 11 et 15 représentent la distribution cumulée en volume pour des particules de verre de différentes tailles enrobées par différents enrobants selon la présente invention ou non enrobées.
Les figures 7 à 10 représentent les images obtenues par MEBE (Microscope Electronique à Balayage Environnemental) de billes de verre micrométriques (50-63 µm) non enrobées (figure 7), enrobées par du Precirol® ATO5 (figures 8 et 10) et un mélange physique de ces billes de verre micrométriques (50-63 µm) avec du Précirol®AT05 (figure 9).
La figure 12 représente les images obtenues par MEBE de billes de verre micrométriques (30-40 µm) enrobées par du Precirol® ATO5.
Les figures 13 représentent la distribution cumulée en volume pour des particules de pseudoephedrine enrobées selon la présente invention ou non enrobées.
La figure 14 représente un test de libération dans l'eau distillée de particules de pseudoéphédrine non enrobées ou enrobées selon la présente invention.
La figure 16 représente les images obtenues par MEBE de billes de verre de granulométrie inférieure à 20 µm enrobées par de la paraffine.
La figure 17 représente les images obtenues par MEBE de billes de verre de granulométrie inférieure à 20 µm enrobées par du monostérate de glycérol.
La figure 18 représente un test de libération en solution de particules de pseudoéphédrine non enrobées ou enrobées selon la présente invention.
La figure 19 représente un test de libération en solution de particules de protéines BSA non enrobées ou enrobées selon la présente invention.

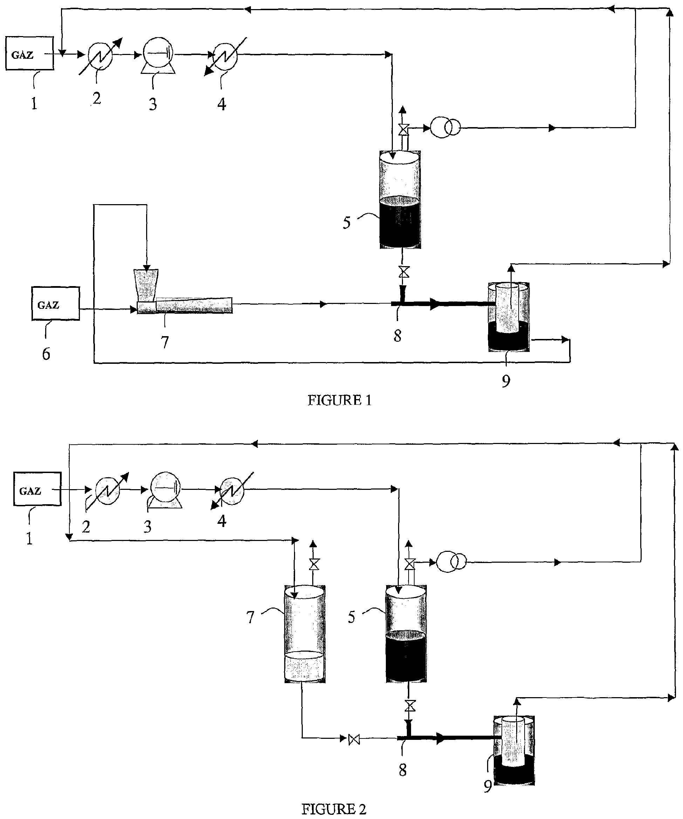

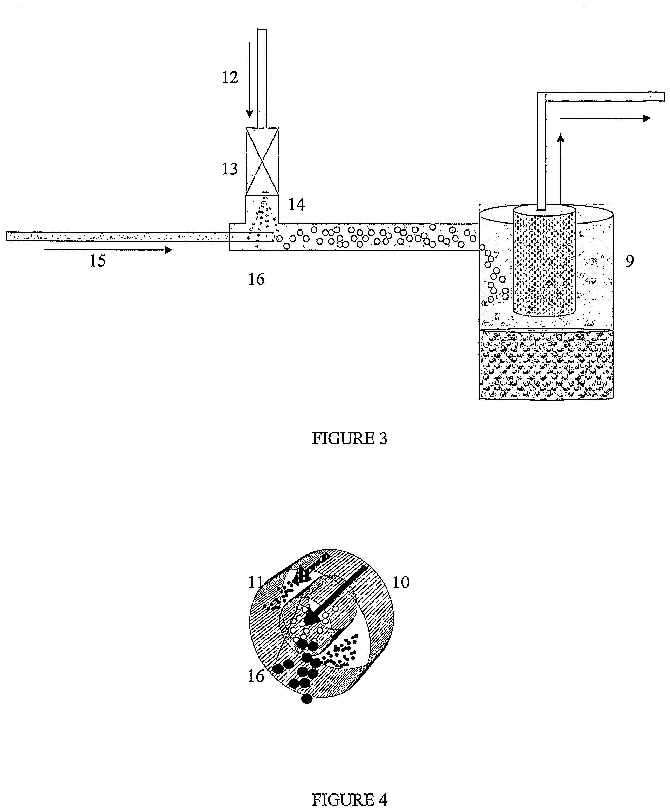

Le mode de mise en oeuvre du procédé représenté sur la figure 1 se déroule de la façon suivante :
Le fluide (CO₂) provenant d'une réserve liquide (1) est refroidi (2) puis pompé (3) et chauffé par un échangeur de chaleur (4). Il devient alors supercritique ou subcritique. On introduit le fluide en conditions supercritiques ou subcritiques dans l'autoclave (5) contenant l'enrobant. La température de l'autoclave est régulée en fonction de la température de fusion de l'enrobant à pression atmosphérique. En général, la saturation de l'enrobant par un gaz comprimé provoque un effondrement de la température de fusion pouvant aller jusqu'à 40 °C en dessous du point de fusion en conditions normales. Ledit fluide se solubilise dans l'enrobant jusqu'à la saturation. Durant cette période la pression est maintenue constante. L'équilibre atteint, le mélange d'enrobant saturé en fluide supercritique ou subcritique ramolli est détendu (8). La perte de charge lors de la détente est comprise de préférence entre 2,03 et 30,38 MPa. Simultanément, la substance active à enrober sous forme de particules solide pulvérulente individualisées en mouvement est envoyée via le dispositif pneumatique (7) et co-injectée de préférence de manière co-axiale avec le mélange enrobant/fluide supercritique ou subcritique détendu (8). Dans cette injection co-axiale, le mélange enrobant/fluide supercritique ou subcritique peut par exemple être à l'extérieur (figure 3). Les particules enrobées sont récupérées dans un filtre de séparation gaz / solide (9), de préférence à pression atmosphérique. Si le CO₂ est le gaz vecteur (6) lors du transport pneumatique, on pourra envisager de le réutiliser en boucle fermée (figure 2).

Les figures 3 et 4 représentent le principe d'une mise en contact par une injection co-axiale des particules individualisées en mouvement de substance active (10) et du mélange enrobant/fluide supercritique ou subcritique (11). On retrouve schématisée en (12) l'arrivée du mélange enrobant / fluide supercritique ou subcritique. Puis ce mélange est détendu (13) et forme ainsi un aérosol (14) qui rejoint le tube extérieur comme exemplifié en (11) (figure 4).
La substance active à enrober est introduite sous forme de particules individualisées en mouvement (15) et rejoint le tube intérieur (10). La mise en contact est effectuée dans l'enceinte (16) où règne une pression suffisante pour permettre le retour du fluide à l'état gazeux et la solidification de l'enrobant autour des particules individualisées de substance active. Les particules enrobées sont enfin collectées dans un filtre de séparation gaz / solide (9).
Ce mode de mise en contact n'est pas exclusif et des variantes ou extensions peuvent être envisagées. Le temps de mise en contact peut par exemple être augmenté en modifiant le trajet du courant transportant le mélange enrobant/fluide supercritique ou subcritique et les particules individualisées en mouvement de substance active à enrober : au lieu d'être linéaire, il peut être hélicoïdal. La mise en contact peut aussi être améliorée par une recirculation des particules individualisées de substance active non enrobées après séparation de celles-ci avec les particules enrobées (figure 1).

Les exemples suivants sont donnés à titre indicatif non limitatif

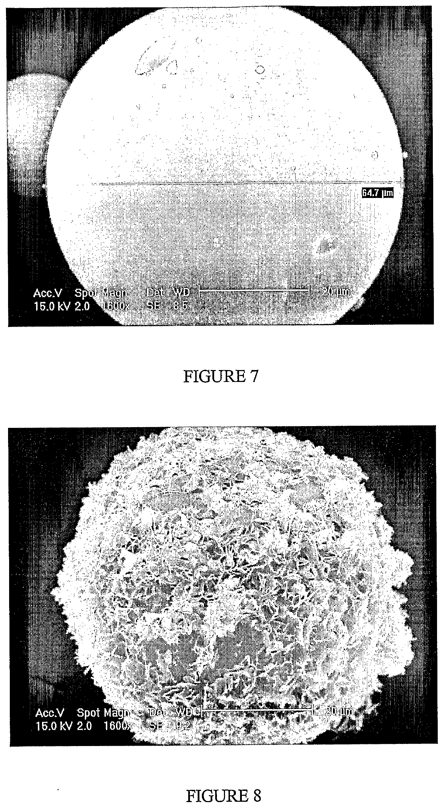

La caractérisation de l'enrobage des particules se fait par granulométrie en voie sèche (appareillage utilisé : Aerosizer® PSD 3603, TSI) qui permet de déterminer la distribution granulométrique à partir du temps de vol des particules dans un courant d'air. Une visualisation des particules enrobées est faite au MEBE, Microscope Electronique à Balayage Environnemental (Philips, XL 30 FEG) (figures 7 à 10, 12, 16 et 17).
Une mise en oeuvre du procédé selon la présente invention peut se faire selon les exemples suivants :

### Exemple 1 :

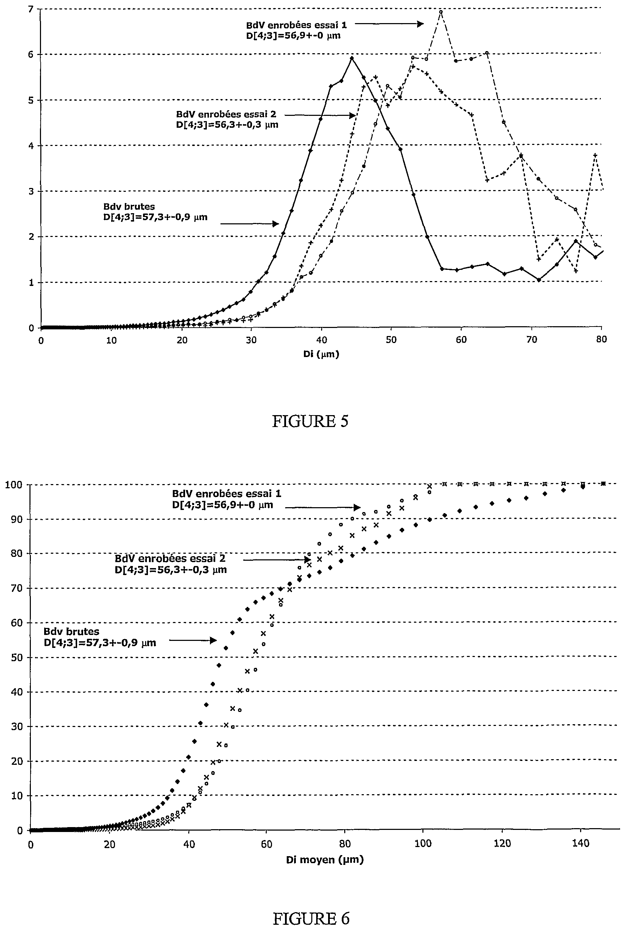

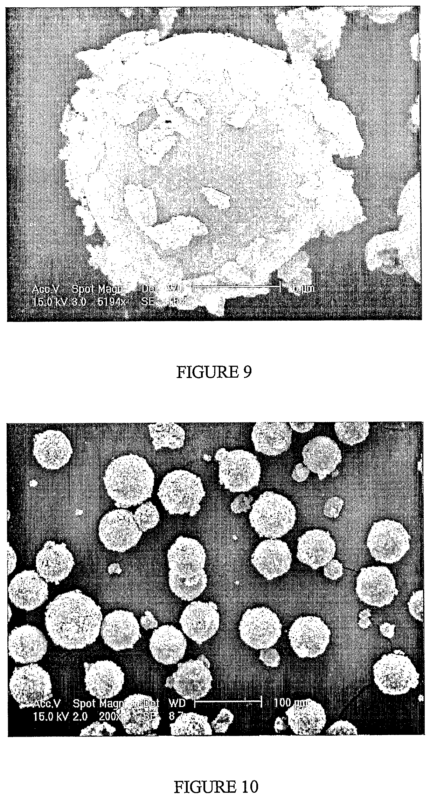

On solubilise du CO₂ supercritique à 50°C et 11,14 MPa dans 3 g de Precirol® ATO5 (fournit par la société Gattefossé) sous forme solide (dipalmitostéarate de glycérol, température de fusion = 56,3°C). La pression dans l'autoclave est maintenue constante. Après 30 minutes, la phase saturée de Precirol® est détendue vers le système de co-injection axiale. Dans le même temps, on co-injecte, selon la configuration de la figure 1, 3 g de billes de verre de granulométrie 50-63 µm par un Venturi dont la pression d'air comprimé atteint 0,51 MPa. Les billes de verre circulent à l'intérieur de deux tubes co-axiaux tandis que le corps gras pulvérisé est admis dans le tube externe. Cette configuration permet un dépôt privilégié de l'enrobant à la surface des particules de verre. L'ensemble est récolté dans un séparateur gaz/solide à pression atmosphérique. Le mélange gazeux CO₂/air comprimé est envoyé à l'évent. Les particules enrobées recueillies sont analysées par granulométrie laser pour contrôler l'augmentation du diamètre moyen après enrobage. On réalise l'expérience deux fois (test 1 et test 2) pour vérifier la reproductibilité des résultats. Le suivi de l'enrobage est contrôlé par granulométrie en utilisant un analyseur de distribution granulométrique PSD3603 (TSI) (figures 5 et 6) et par image MEBE (figures 7, 8, 9 et 10) (Microscope Electronique à Balayage Environnemental). Un revêtement représentatif du Précirol® sur une bille de verre obtenu par le procédé selon la présente invention est montré à la figure 8. L'enrobage de la figure 8 est homogène et diffère complètement du simple mélange physique des deux composants (figure 9) et de l'aspect de surface d'une bille de verre brute (figure 7). La figure 10 montre un ensemble de particules obtenues par enrobage selon la présente invention. Cet ensemble montre l'homogénéité de l'échantillon des particules enrobées.
Ceci montre que le revêtement n'est pas seulement un dépôt de Precirol® solide sur les billes de verre mais implique un phénomène de solidification/dépôt sur la surface de la bille. Les figures 5 et 6 permettent de constater un décalage du diamètre géométrique moyen vers les diamètres plus élevés, des particules enrobées par rapport aux particules non enrobées. Cette croissance du diamètre moyen des particules est imputable au dépôt d'une couche d'enrobant à la surface des particules.
En outre les deux courbes granulométriques sur les particules enrobées des essais 1 et 2 et visibles sur les figures 5 et 6 sont similaires et permettent de déduire une très bonne reproductibilité du procédé.

### Exemple 2 :

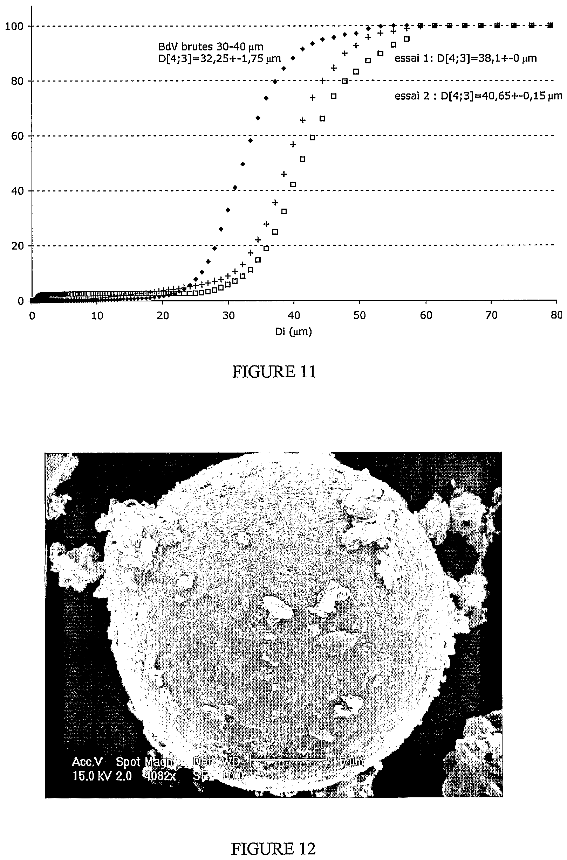

Dans cet exemple, on change les masses initiales d'enrobé et d'enrobant. On solubilise, dans les mêmes conditions que dans l'exemple 1, 4 g de corps gras Precirol® ATO5 sous forme solide mais on restreint la granulométrie des billes de verre à une taille 30-40 µm. On diminue la quantité d'enrobant employée pour l'enrobage. En outre, on utilise du CO₂ gazeux pour convoyer les 6 g de billes de verre vers le système de co-injection (selon la figure 2). Les particules sont recueillies dans le collecteur à pression atmosphérique. On réalise l'expérience deux fois (test 1 et test 2) pour vérifier la reproductibilité des résultats. Le suivi de l'enrobage est contrôlé par granulométrie en utilisant un analyseur de distribution granulométrique PSD3603 (TSI) (figure 11) et par image MEBE (Microscope Electronique à Balayage Environnemental) (figure 12). La figure 11 permet de constater un décalage du diamètre moyen des particules vers des diamètres plus élevés. L'augmentation du diamètre des particules enrobées par rapport au diamètre des particules non enrobées est une conséquence du dépôt d'enrobant à la surface des particules. La figure 12 est une photographie MEBE représentative de l'ensemble obtenu, d'une bille de verre enrobée par le Precirol®.

### Exemple 3 :

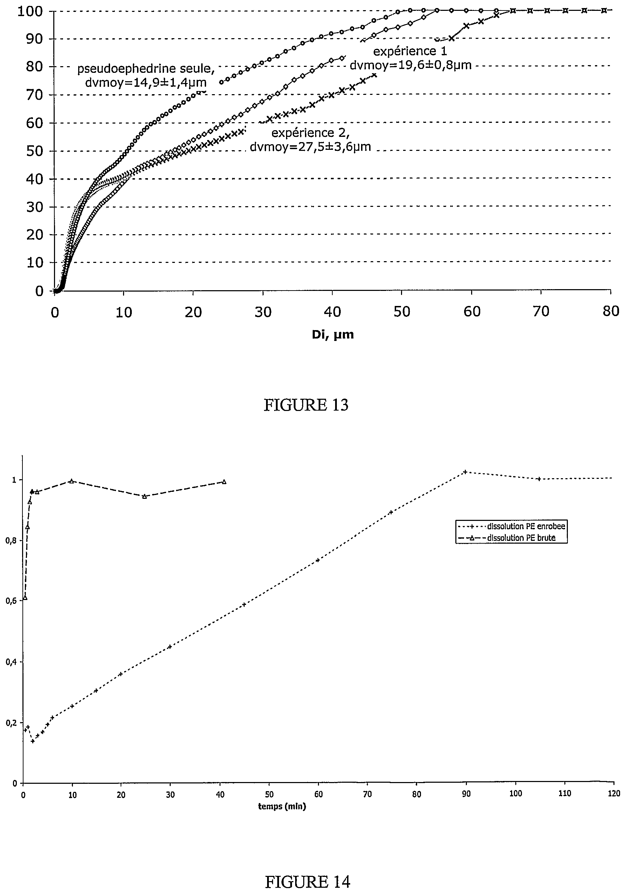

On solubilise du CO₂ supercritique à 50°C et 11,14 MPa dans 3 g de Precirol® ATO5 sous forme solide (dipalmitostéarate de glycérol, température de fusion = 56,3°C). La pression dans l'autoclave est maintenue constante. Après 30 minutes, le Precirol® ATO5 saturé en CO₂ est détendu vers le système de co-injection axiale. Dans le même temps, on co-injecte, selon la configuration de la figure 2, 3 g de particules de pseudoéphédrine de diamètre moyen en volume égal à 14,9±1,4µm. La pression dans l'autoclave via lequel se fait l'injection de la pseudoéphédrine est fixée à 5,06 MPa. Elles circulent à l'intérieur du tube central de l'injection co-axiale tandis que le corps gras pulvérisé est admis dans le tube externe. L'ensemble est récolté dans un séparateur gaz/solide à pression atmosphérique. On réalise l'expérience deux fois pour vérifier la reproductibilité des résultats (tests 1 et 2). Les particules enrobées recueillies sont analysées par granulométrie laser en utilisant un analyseur de distribution granulométrique PSD3603 (TSI) pour contrôler l'augmentation du diamètre moyen après enrobage. On donne à la figure 13 les distributions cumulées en volume de la pseudoéphédrine seule et de la pseudoéphédrine enrobée. On voit nettement le déplacement de la distribution vers les tailles plus élevées, phénomène dû à l'enrobage et à l'agglomération des particules.

Les tests de dissolution réalisés dans de l'eau distillée à température ambiante montrent que le revêtement est efficace puisqu'un délai marqué est observé dans la libération du composé actif (figure 14). Les particules enrobées présentent un retard à la libération par rapport à de la pseudoephédrine non enrobée.

### Exemple 4

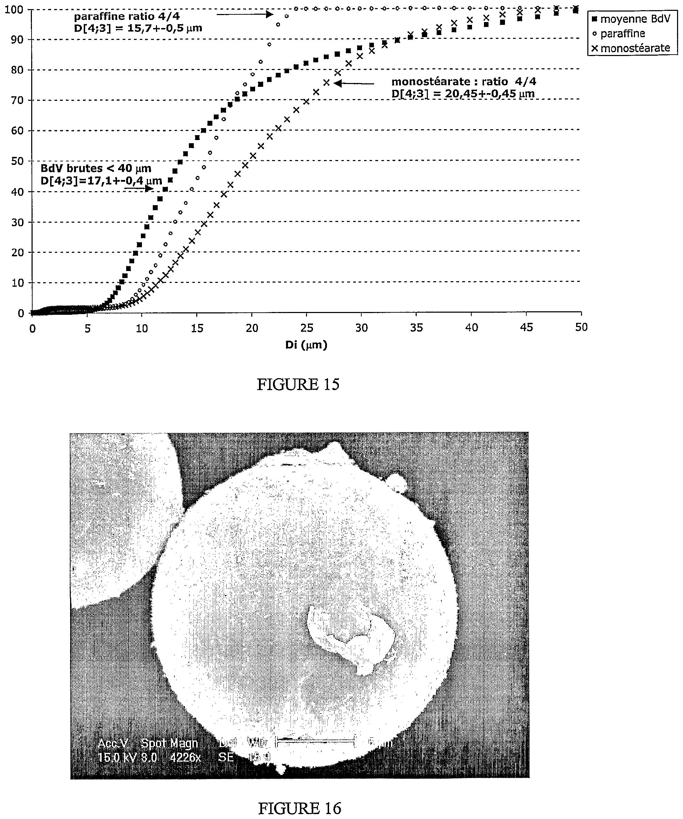

On réalise cette fois ci l'enrobage de particules de bille de verre dont la granulométrie est inférieure à 20 µm. On dissout du CO₂ supercritique dans les conditions de pression 11,14 MPa et de température 53°C, dans 4 g de Paraffine utilisée pour de l'enrobage de composés pharmaceutiques. On utilise du CO₂ gazeux à la pression de 8 MPa pour convoyer les 6 g de billes de verre vers le système de co-injection (selon la figure 2). Après un temps de dissolution de 30 min, les particules non enrobées sont véhiculées par le CO₂ gazeux et simultanément la paraffine est cristallisée par ouverture de la vanne en sortie de l'enceinte de dissolution. Les billes de verre circulent à l'intérieur de deux tubes co-axiaux alors que le corps gras pulvérisé est véhiculé dans le tube externe. Les particules sont recueillies dans le collecteur à pression atmosphérique. Le suivi de l'enrobage est contrôlé par granulométrie en utilisant un analyseur de distribution granulométrique PSD3603 (TSI) (figure 15) et par image MEBE (Microscope Electronique à Balayage Environnemental) (figure 16). La figure 15 permet de constater un décalage du diamètre moyen des particules vers des diamètres plus élevés. L'augmentation du diamètre des particules enrobées par rapport au diamètre des particules non enrobées est une conséquence du dépôt d'enrobant à la surface des particules. La figure 16 permet de visualiser l'enrobage d'une particule représentative de bille de verre. On aperçoit une couche continue d'enrobant à la surface de la bille de verre. Cet exemple montre que le procédé est applicable à des particules d'une granulométrie inférieure à 20 µm.

### Exemple 5

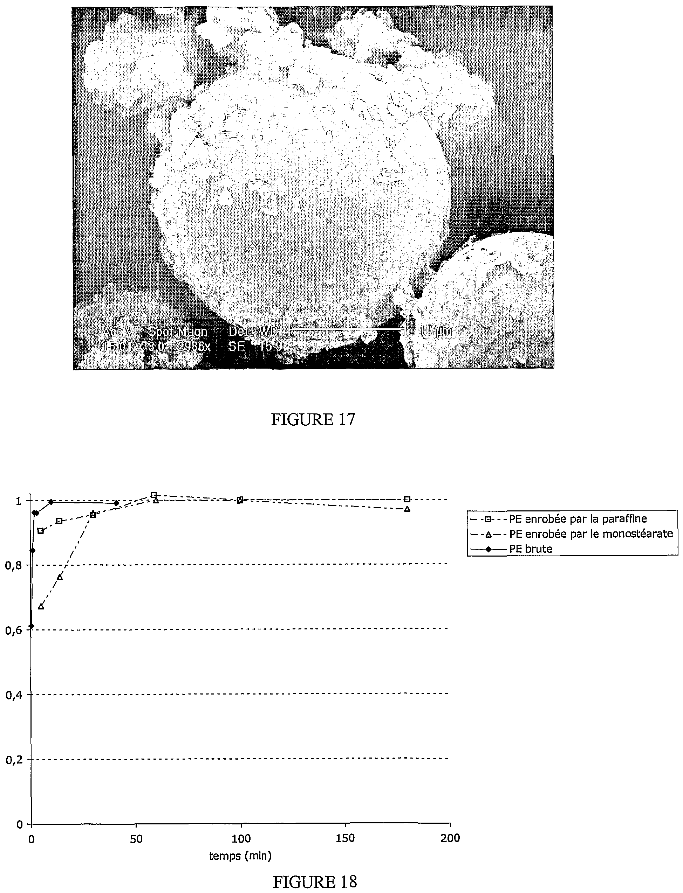

On réalise de nouveau l'enrobage de particules de de bille de verre dont la granulométrie est inférieure à 20 µm. Cette fois-ci, on dissout du CO₂ supercritique dans les conditions de pression 11,14 MPa et de température 57,3°C, dans 4 g de monostéarate de glycérol utilisé à titre d'agent d'enrobage dans l'industrie pharmaceutique. On utilise du CO₂ gazeux à la pression de 8 MPa pour convoyer les 6 g de billes de verre vers le système de co-injection (selon la figure 2). La mise en oeuvre est similaire à celle de l'exemple 4. Le suivi de l'enrobage est contrôlé par granulométrie en utilisant un analyseur de distribution granulométrique PSD3603 (TSI) (figure 15) et par image MEBE (Microscope Electronique à Balayage Environnemental) (figure 17). La figure 15 permet de constater un décalage du diamètre moyen des particules enrobées par le monostéarate vers des diamètres plus élevés. La figure 17 permet de visualiser la couche de dépôt d'enrobant à la surface des particules de bille de verre.

### Exemple 6

Le procédé revendiqué est mis en oeuvre selon la figure 2 pour enrober de la pseudoéphedrine avec du monostéarate de glycérol, dont la température de fusion à pression atmosphérique est 60,3°C. On solubilise le CO₂ supercritique à la température de 57°C et à la pression de 110 bars dans 4 g du corps gras dédié à l'enrobage. Après un temps d'équilibre de 30 minutes, la phase lourde de corps gras liquéfié et de CO₂ dissous est détendue. Simultanément 4 g de pseudoephedrine sont injectés par du CO₂ à la pression de 80 bars. Les particules sont collectées dans le séparateur solide/gaz pour analyse. La figure 18 présente le dosage par spectrophotomètrie UV de la pseudoephedrine enrobée libérée dans de l'eau distillée à température ambiante. La pseudoephedrine brute est entièrement dissoute en solution en 10 minutes. La pseudoephedrine présente dans les particules enrobées met 60 minutes pour se dissoudre en milieu aqueux, soit un retard de près de 50 minutes à la dissolution.

### Exemple 7

L'exemple 7 présente l'enrobage de 4 g de pseudoephedrine par 4 g de paraffine dont le point de fusion à pression atmosphérique est de 58,5°C. Les conditions de solubilisation du CO₂ supercritique dans le corps gras sont de 110 bars et 55°C. Le temps de dissolution est de 30 minutes. Après l'étape de dissolution, les deux courants enrobé et enrobant sont co-injectés, selon la figure 2 du procédé selon la présente invention. Les particules enrobées sont dosées par spectrophotomètrie UV pour repérer l'effet retard à la dissolution causé par l'enrobage des particules (Figure 18). On remarque sur la figure 18 que la dissolution dans de l'eau distillée à température ambiante du principe actif est retardée d'environ 50 minutes par l'enrobage par rapport à la pseudoephédrine brute.

### Exemple 8

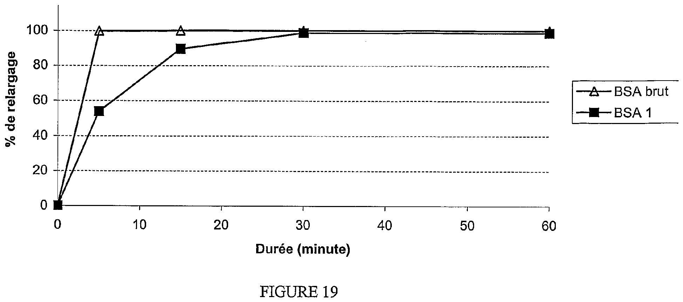

On enrobe cette fois ci selon la figure 2 du procédé selon de la présente invention, une protéine thermosensible, la BSA (albumine de sérum bovin Bovine Serum Albumin) dont le diamètre moyen mesuré par granulométrie est de 85,6 µm. On place 3 g de BSA dans le système d'injection par le CO₂ et 3 g de Precirol® dans l'autoclave. Les conditions en milieu supercritique sont de 110 bars et 50°C pour un temps de solubilisation de 30 minutes. La pression d'injection des particules de BSA est de 80 bars. Une fois la co-injection réalisée, les particules enrobées sont dosées par spectrophotométrie UV (Figure 19). Tandis que la BSA brute est entièrement dissoute dans de l'eau distillée à température ambiante au bout de 5 minutes, la protéine enrobée par le Precirol® (essai BSA 1) met quant à elle près de 30 minutes pour être entièrement solubilisée dans de l'eau distillée à température ambiante. la surface des particules. La figure 16 permet de visualiser l'enrobage d'une particule représentative de bille de verre. On aperçoit une couche continue d'enrobant à la surface de la bille de verre. Cet exemple montre que le procédé est applicable à des particules d'une granulométrie inférieure à 20 µm.

### Exemple 5

On réalise de nouveau l'enrobage de particules de de bille de verre dont la granulométrie est inférieure à 20 µm. Cette fois-ci, on dissout du CO₂ supercritique dans les conditions de pression 11,14 MPa et de température 57,3°C, dans 4 g de monostéarate de glycérol utilisé à titre d'agent d'enrobage dans l'industrie pharmaceutique. On utilise du CO₂ gazeux à la pression de 8 MPa pour convoyer les 6 g de billes de verre vers le système de co-injection (selon la figure 2). La mise en oeuvre est similaire à celle de l'exemple 4. Le suivi de l'enrobage est contrôlé par granulométrie en utilisant un analyseur de distribution granulométrique PSD3603 (TSI) (figure 15) et par image MEBE (Microscope Electronique à Balayage Environnemental) (figure 17). La figure 15 permet de constater un décalage du diamètre moyen des particules enrobées par le monostéarate vers des diamètres plus élevés. La figure 17 permet de visualiser la couche de dépôt d'enrobant à la surface des particules de bille de verre.

### Exemple 6

Le procédé revendiqué est mis en oeuvre selon la figure 2 pour enrober de la pseudoéphedrine avec du monostéarate de glycérol, dont la température de fusion à pression atmosphérique est 60,3°C. On solubilise le CO₂ supercritique à la température de 57°C et à la pression de 110 bars dans 4 g du corps gras dédié à l'enrobage. Après un temps d'équilibre de 30 minutes, la phase lourde de corps gras liquéfié et de CO₂ dissous est détendue. Simultanément 4 g de pseudoephedrine sont injectés par du CO₂ à la pression de 80 bars. Les particules sont collectées dans le séparateur solide/gaz pour analyse. La figure 18 présente le dosage par spectrophotomètrie UV de la pseudoephedrine enrobée libérée dans de l'eau distillée à température ambiante. La pseudoephedrine brute est entièrement dissoute en solution en 10 minutes. La pseudoephedrine présente dans les particules enrobées met 60 minutes pour se dissoudre en milieu aqueux, soit un retard de près de 50 minutes à la dissolution.

### Exemple 7

L'exemple 7 présente l'enrobage de 4 g de pseudoephedrine par 4 g de paraffine dont le point de fusion à pression atmosphérique est de 58,5°C. Les conditions de solubilisation du CO₂ supercritique dans le corps gras sont de 110 bars et 55°C. Le temps de dissolution est de 30 minutes. Après l'étape de dissolution, les deux courants enrobé et enrobant sont co-injectés, selon la figure 2 du procédé selon la présente invention. Les particules enrobées sont dosées par spectrophotomètrie UV pour repérer l'effet retard à la dissolution causé par l'enrobage des particules (Figure 18). On remarque sur la figure 18 que la dissolution dans de l'eau distillée à température ambiante du principe actif est retardée d'environ 50 minutes par l'enrobage par rapport à la pseudoephédrine brute.

### Exemple 8

On enrobe cette fois ci selon la figure 2 du procédé selon de la présente invention, une protéine thermosensible, la BSA (albumine de sérum bovin Bovine Serum Albumin) dont le diamètre moyen mesuré par granulométrie est de 85,6 µm. On place 3 g de BSA dans le système d'injection par le CO₂ et 3 g de Precirol® dans l'autoclave. Les conditions en milieu supercritique sont de 110 bars et 50°C pour un temps de solubilisation de 30 minutes. La pression d'injection des particules de BSA est de 80 bars. Une fois la co-injection réalisée, les particules enrobées sont dosées par spectrophotométrie UV (Figure 19). Tandis que la BSA brute est entièrement dissoute dans de l'eau distillée à température ambiante au bout de 5 minutes, la protéine enrobée par le Precirol® (essai BSA 1) met quant à elle près de 30 minutes pour être entièrement solubilisée dans de l'eau distillée à température ambiante.

## Revendications

1. Procédé d'enrobage de substances actives solides pulvérulentes **caractérisé en ce qu'**il comprend les étapes suivantes :
a) fournir un mélange comprenant au moins un enrobant dans lequel est dissous jusqu'à saturation un fluide supercritique ou subcritique dans des conditions de température et de pression permettant le maintien dudit fluide dans des conditions supercritiques ou subcritiques ;
b) fournir des particules individualisées en mouvement d'au moins une substance active solide pulvérulente ;
c) mettre en contact le mélange et les particules individualisées en mouvement de substance active dans des conditions de température et de pression assurant simultanément :
- la détente du fluide supercritique ou subcritique et son retour à l'état gazeux,
- la pulvérisation de l'enrobant et
- sa solidification autour des particules individualisées en mouvement de substance active ;
d) séparer et récupérer les particules enrobées,
ledit fluide supercritique ou subcritique étant le CO₂.

2. Procédé d'enrobage selon la revendication 1 caractérisé en ce l'étape (b) comprend en outre l'ajout aux particules individualisées en mouvement de substance active d'un solvant organique choisi dans le groupe comprenant les cétones, alcools, esters, alcanes, alcènes ou un mélange de ceux-ci.

3. Procédé d'enrobage selon la revendication 1 ou 2 **caractérisé en ce que** l'étape (a) comprend en outre l'ajout au mélange d'un solvant organique choisi dans le groupe comprenant les cétones, alcools, esters, alcanes, alcènes ou un mélange de ceux-ci.

4. Procédé d'enrobage selon les revendications 1 à 3 **caractérisé en ce que** l'enrobant est choisi dans le groupe comprenant les polysaccharides et leurs dérivés, les polymères synthétiques de type acryliques ou méthacryliques, les lipides, les phospholipides ou un mélange de ceux-ci.

5. Procédé d'enrobage selon les revendications 1 à 4 **caractérisé en ce que** l'étape (a) comprend en outre l'ajout au mélange d'un agent plastifiant.

6. Procédé d'enrobage selon les revendications 1 à 5 **caractérisé en ce que** la taille moyenne des particules individualisées de substance active de l'étape (b) est inférieure à 100µm, avantageusement inférieure à 50µm.

7. Procédé d'enrobage selon les revendications 1 à 6 **caractérisé en ce que** la substance active est thermosensible.

8. Procédé d'enrobage selon les revendications 1 à 7 **caractérisé en ce que** le ratio massique substance active / enrobant est compris entre 10/1 et 1/10, préférentiellement entre 5/1 et 1/5.

9. Procédé d'enrobage selon les revendications 1 à 8 **caractérisé en ce que** l'étape (c) de mise en contact du mélange avec les particules individualisées de substance active est réalisée dans un courant transporteur obtenu par co-injection.

10. Procédé d'enrobage selon la revendication 9 **caractérisé en ce que** le courant transporteur est linéaire ou hélicoïdal.

11. Procédé d'enrobage selon les revendications 1 à 10 **caractérisé en ce que** l'étape (b) consiste en le convoyage des particules individualisées de substance active par transport pneumatique ou mécanique avec du CO₂ ou de l'air comprimé.

12. Procédé d'enrobage selon les revendications 1 à 11 **caractérisé en ce que** la substance active est choisie dans le groupe des substances actives pharmaceutiques, cosmétiques, nutraceutiques, alimentaires, agrochimiques, vétérinaires et leur mélange.

13. Microparticules de substance active enrobées, chaque microparticule comprenant successivement un noyau comprenant la substance active et une écorce comprenant l'enrobant, **caractérisées**
- **en ce qu'**elles sont susceptibles d'être obtenues par le procédé selon les revendications 1 à 2 et 4 à 12, ledit fluide supercritique ou subcritique étant le CO₂,
- **en ce que** la substance active est solide à température ambiante et thermosensible, de préférence instable à une température supérieure à 28°C, et
- **en ce que** la taille moyenne du noyau de substance active est inférieure à 50µm.

14. Microparticules selon la revendication 13, **caractérisées**
- **en ce que** l'enrobant est solide à température ambiante et n'est pas soluble dans le fluide supercritique ou subcritique, et
- **en ce que** les particules enrobées sont exemptes de solvant organique.

15. Microparticules selon les revendications 13 ou 14 **caractérisées en ce que** l'enrobant permet de masquer le goût et/ou de la couleur de la substance active.

16. Microparticules selon les revendications 13 ou 14 **caractérisées en ce que** l'enrobant permet une libération contrôlée de la substance active et une augmentation de sa biodisponibilité.

17. Microparticules selon les revendications 13 ou 14 **caractérisées en ce que** l'enrobant permet de protéger la substance active vis à vis d'agents externes de dégradation.

18. Microparticules selon la revendication 16 **caractérisées en ce que** la substance active est chemosensible ou photosensible.

19. Utilisation des microparticules selon les revendications 13 à 18 dans une formulation orale, topique, injectable ou rectale.

20. Utilisation des microparticules selon les revendications 13 à 18 dans des formulations pharmaceutiques, cosmétiques, nutraceutiques, alimentaires, agrochimiques ou vétérinaires.

## Patentansprüche

1. Verfahren zum Überziehen von festen pulverförmigen Wirkstoffen, **dadurch gekennzeichnet, dass** es die folgenden Schritte umfasst:
a) Bereitstellen einer mindestens ein Überzugsmittel umfassenden Mischung, in der bis Sättigung ein überkritisches oder unterkritisches Fluid gelöst ist, unter Temperatur- und Druckbedingungen, die das Halten des Fluids in überkritischen oder unterkritischen Bedingungen ermöglichen;
b) Bereitstellen von vereinzelten, sich in Bewegung befindlichen Teilchen mindestens eines festen pulverförmigen Wirkstoffs;
c) Inkontaktbringen der Mischung und der vereinzelten, sich in Bewegung befindlichen Wirkstoffteilchen unter Temperatur- und Druckbedingungen, die gleichzeitig sicherstellen:
- das Entspannen des überkritischen oder unterkritischen Fluids und seine Rückkehr in den gasförmigen Zustand,
- das Zerstäuben des Überzugsmittels, und
- sein Verfestigen um die vereinzelten, sich in Bewegung befindlichen Wirkstoffteilchen herum;
d) Abscheiden und Gewinnen der überzogenen Teilchen,
wobei das überkritische oder unterkritische Fluid CO₂ ist.

2. Überzugsverfahren nach Anspruch 1, **dadurch gekennzeichnet, dass** der Schritt (b) weiter das Zugeben eines organischen Lösungsmittels zu den vereinzelten, sich in Bewegung befindlichen Wirkstoffteilchen umfasst, ausgewählt aus der Gruppe, die Ketone, Alkohole, Ester, Alkane, Alkene oder eine Mischung derselben umfasst.

3. Überzugsverfahren nach Anspruch 1 oder 2, **dadurch gekennzeichnet, dass** der Schritt (a) weiter das Zugeben eines organischen Lösungsmittels zur Mischung umfasst, ausgewählt aus der Gruppe, die Ketone, Alkohole, Ester, Alkane, Alkene oder eine Mischung derselben umfasst.

4. Überzugsverfahren nach den Ansprüchen 1 bis 3, **dadurch gekennzeichnet, dass** das Überzugsmittel aus der Gruppe ausgewählt ist, die Polysaccharide und deren Derivate, synthetische Polymere vom Acryl- oder Methacryltyp, Lipide, Phospholipide oder eine Mischung derselben umfasst.

5. Überzugsverfahren nach den Ansprüchen 1 bis 4, **dadurch gekennzeichnet, dass** der Schritt (a) weiter das Zugeben eines Weichmachers zur Mischung umfasst.

6. Überzugsverfahren nach den Ansprüchen 1 bis 5, **dadurch gekennzeichnet, dass** die mittlere Größe der vereinzelten Wirkstoffteilchen aus dem Schritt (b) weniger als 100 µm, vorteilhafterweise weniger als 50 µm beträgt.

7. Überzugsverfahren nach den Ansprüchen 1 bis 6, **dadurch gekennzeichnet, dass** der Wirkstoff wärmeempfindlich ist.

8. Überzugsverfahren nach den Ansprüchen 1 bis 7, **dadurch gekennzeichnet, dass** das Massenverhältnis Wirkstoff / Überzugsmittel im Bereich zwischen 10/1 und 1/10, vorzugsweise zwischen 5/1 und 1/5 beträgt.

9. Überzugsverfahren nach den Ansprüchen 1 bis 8, **dadurch gekennzeichnet, dass** der Schritt (c) des Inkontaktbringens der Mischung mit den vereinzelten Wirkstoffteilchen in einem Transportstrom ausgeführt wird, der durch Koinjektion erhalten wird.

10. Überzugsverfahren nach Anspruch 9, **dadurch gekennzeichnet, dass** der Transportstrom gerade oder spiralförmig ist.

11. Überzugsverfahren nach den Ansprüchen 1 bis 10, **dadurch gekennzeichnet, dass** der Schritt (b) im Befördern der vereinzelten Wirkstoffteilchen durch pneumatischen oder mechanischen Transport mit CO₂ oder Druckluft besteht.

12. Überzugsverfahren nach den Ansprüchen 1 bis 11, **dadurch gekennzeichnet, dass** der Wirkstoff aus der Gruppe der pharmazeutischen, kosmetischen, nutrizeutischen, alimentären, agrochemischen, veterinären Wirkstoffe und deren Mischung ausgewählt ist.

13. Überzogene Wirkstoff-Mikroteilchen, wobei jedes Mikroteilchen nacheinander einen den Wirkstoff umfassenden Kern und eine das Überzugsmittel umfassende Hülle umfasst, **gekennzeichnet**
- **dadurch, dass** sie über das Verfahren nach den Ansprüchen 1 bis 2 und 4 bis 12 erhalten werden können, wobei das überkritische oder unterkritische Fluid CO₂ ist,
- dadurch, dass der Wirkstoff bei Umgebungstemperatur fest und wärmeempfindlich, vorzugsweise bei einer Temperatur über 28 °C instabil ist, und
- dadurch, dass die mittlere Größe des Wirkstoffkerns weniger als 50 µm beträgt.

14. Mikroteilchen nach Anspruch 13, **gekennzeichnet**
- **dadurch, dass** das Überzugsmittel bei Umgebungstemperatur fest ist und nicht im überkritischen oder unterkritischen Fluid löslich ist, und
- dadurch, dass die überzogenen Teilchen frei von organischem Lösungsmittel sind.

15. Mikroteilchen nach den Ansprüchen 13 oder 14, **dadurch gekennzeichnet, dass** das Überzugsmittel ermöglicht, den Geschmack und/oder die Farbe des Wirkstoffs zu kaschieren.

16. Mikroteilchen nach den Ansprüchen 13 oder 14, **dadurch gekennzeichnet, dass** das Überzugsmittel eine kontrollierte Freisetzung des Wirkstoffs und eine Steigerung seiner Bioverfügbarkeit ermöglicht.

17. Mikroteilchen nach den Ansprüchen 13 oder 14, **dadurch gekennzeichnet, dass** das Überzugsmittel ermöglicht, den Wirkstoff vor äußeren Abbaumitteln zu schützen.

18. Mikroteilchen nach Anspruch 16, **dadurch gekennzeichnet, dass** der Wirkstoff chemosensitiv oder lichtempfindlich ist.

19. Verwendung der Mikroteilchen nach den Ansprüchen 13 bis 18 in einer oralen, topischen, injizierbaren oder rektalen Formulierung.

20. Verwendung der Mikroteilchen nach den Ansprüchen 13 bis 18 in pharmazeutischen, kosmetischen, nutrizeutischen, alimentären, agrochemischen oder veterinären Formulierungen.

## Claims

1. Process for coating pulverulent solid active substances, **characterized in that** it comprises the following stages:
a) providing a mixture comprising at least one coating agent in which is dissolved, up to saturation, a supercritical or subcritical fluid under temperature and pressure conditions which make it possible to maintain the said fluid under supercritical or subcritical conditions;
b) providing separate moving particles of at least one pulverulent solid active substance;
c) bringing the mixture and the separate moving particles of active substance into contact under temperature and pressure conditions which simultaneously ensure:
- the reduction in pressure of the supercritical or subcritical fluid and its return to the gaseous state,
- the spraying of the coating agent and
- its solidification around the separate moving particles of active substance;
d) separating and recovering the coated particles. said supercritical fluid or subcritical fluid being CO₂.

2. Coating process according to Claim 1, **characterized in that** stage (b) additionally comprises the addition, to the separate moving particles of active substance, of an organic solvent chosen from the group comprising ketones, alcohols, esters, alkanes, alkenes and a mixture of these.

3. Coating process according to Claim 1 or 2, **characterized in that** stage (a) additionally comprises the addition, to the mixture, of an organic solvent chosen from the group comprising ketones, alcohols, esters, alkanes, alkenes and a mixture of these.

4. Coating process according to Claims 1 to 3, **characterized in that** the coating agent is chosen from the group comprising polysaccharides and their derivatives, synthetic polymers of acrylic or methacrylic type, lipids, phospholipids and a mixture of these.

5. Coating process according to Claims 1 to 4, **characterized in that** stage (a) additionally comprises the addition to the mixture of a plasticizing agent.

6. Coating process according to Claims 1 to 5, **characterized in that** the mean size of the separate particles of active substance of stage (b) is less than 100 µm, advantageously less than 50 µm.

7. Coating process according to Claims 1 to 6, **characterized in that** the active substance is heat-sensitive.

8. Coating process according to Claims 1 to 7, **characterized in that** the active substance/coating agent ratio by weight is between 10/1 and 1/10, preferably between 5/1 and 1/5.

9. Coating process according to Claims 1 to 8, **characterized in that** stage (c) of bringing the mixture into contact with the separate particles of active substance is carried out in a feed stream obtained by coinjection.

10. Coating process according to Claim 9, **characterized in that** the feed stream is linear or helical.

11. Coating process according to Claims 1 to 10, **characterized in that** stage (b) consists of the conveying of the separate particles of active substance by pneumatic or mechanical transportation with CO₂ or compressed air.

12. Coating process according to Claims 1 to 11, **characterized in that** the active substance is chosen from the group of pharmaceutical, cosmetic, nutraceutic, food, agrochemical and veterinary active substances and their mixture.

13. Coated microparticles of active substance, each microparticle successively comprising a core comprising the active substance and a shell comprising the coating agent, **characterized**
- **in that** they are capable of being obtained by the process according to Claims 1 to 2 and 4 to 12, said supercritical fluid or subcritical fluid being CO₂.
- **in that** the active substance is solid at ambient temperature and is sensitive to heat, preferably unstable at a temperature greater than 28°C, and
- **in that** the mean size of the core of active substance is less than 50 µm.

14. Microparticles according to Claim 13, **characterized**
- **in that** the coating agent is solid at ambient temperature and is insoluble in the supercritical or subcritical fluid, and
- **in that** the coated particles are devoid of organic solvent.

15. Microparticles according to Claims 13 or 14, **characterized in that** the coating agent makes it possible to mask the taste and/or color of the active substance.

16. Microparticles according to Claims 13 or 14, **characterized in that** the coating agent makes possible controlled release of the active substance and an increase in its bioavailability.

17. Microparticles according to Claims 13 or 14, **characterized in that** the coating agent makes it possible to protect the active substance with regard to external agents which cause damage.

18. Microparticles according to Claim 16, **characterized in that** the active substance is sensitive to chemicals or to light.

19. Use of the microparticles according to Claims 13 to 18 in an oral, topical, injectable or rectal formulation.

20. Use of the particles according to Claims 13 to 18 in pharmaceutical, cosmetic, nutraceutic, food, agrochemical or veterinary formulations.
